# FASCICULE DE BREVET EUROPEEN

(11) **EP 4 153 981 B1**
(45) Date de publication et mention de la délivrance du brevet: **02.12.2026**
(21) Numéro de dépôt: 21727348.1
(22) Date de dépôt: 19.05.2021
(51) Int. Cl.: G01N 27/02, G01N 33/02, G01N 33/12

(54) **PROCEDE DE MESURE D'UNE CONCENTRATION D'AU MOINS UN SEL ET/OU D'AU MOINS UN SOLUTE**
VERFAHREN ZUR MESSUNG EINER KONZENTRATION VON MINDESTENS EINEM SALZ UND/ODER MINDESTENS EINEM GELÖSTEN STOFF
METHOD FOR MEASURING A CONCENTRATION OF AT LEAST ONE SALT AND/OR OF AT LEAST ONE SOLUTE

(30) Priorité: 21.05.2020 FR 2005400
(43) Date de publication de la demande: 29.03.2023
(73) Titulaire: Olen, 29900 Concarneau (FR)
(72) Inventeur: BOULBRY, Bruno, 56100 LORIENT (FR); BODILIS, Gildas, 29900 CONCARNEAU (FR)
(74) Mandataire: IPAZ
(86) Numéro de dépôt international: PCT/EP2021/063241
(87) Numéro de publication internationale: WO 2021/233969

(56) Documents cités:
- CN-B- 105 021 663
- JP-A- S58 198 749
- KR-A- 20160 075 396
- KR-A- 20160 075 396
- US-B1- 7 998 513

## Description

### Domaine technique

La présente invention concerne un procédé de mesure d'une concentration d'au moins un sel et/ou d'au moins un soluté. Elle concerne aussi un dispositif de mesure d'une concentration d'au moins un sel et/ou d'au moins un soluté, et un navire ou bateau ou installation à terre ou usine comprenant un tel dispositif.

Le domaine de l'invention est plus particulièrement, mais de manière non limitative, celui de la conservation, congélation et réfrigération de produits halieutiques ; l'invention peut également s'appliquer à d'autres secteurs de l'agro-alimentaire utilisant la saumure comme méthode de conservation ou de salaison.

### Etat de la technique antérieure

Des techniques de congélation sont nécessaires à bord des navires de pêche congélateurs afin de préserver la qualité du poisson. En effet, les navires partent en mer plusieurs semaines, ce qui nécessite de conserver les poissons pour une longue période, afin qu'ils soient commercialisables et consommables à leur débarquement.

De la même manière, les techniques de refroidissement et de congélation s'appliquent à une large variété de produits alimentaires sous forme solide : poissons, viandes, fruits et légumes. Les techniques de congélation rapide ou de surgélation présentent de nombreux avantages : ce procédé limite la formation de cristaux de grosse taille dans les chairs, limite la diffusion de sel par osmose au coeur des produits et la perte d'exsudat lors de la phase de décongélation.

Le processus de congélation réduit rapidement les modifications physiques, biochimiques et microbiologiques dans le produit. Il résulte de mécanismes de transfert thermique distincts : un transfert convectif entre le produit à congeler et le fluide de refroidissement (le plus souvent l'air, la saumure dans notre cas), et un transfert conductif dans le produit lui-même. Le temps requis pour la congélation du produit est déterminé comme suit : quand le coefficient de transfert convectif est faible, on peut généralement ignorer le temps de conduction, alors qu'à fort coefficient de transfert le terme conductif devient le facteur limitant.

L'eau de mer et la saumure sont de bons moyens de conserver et de congeler des produits halieutiques ou agro-alimentaires grâce à leur faible coût (la saumure n'est autre que de l'eau de mer dans laquelle on vient diluer du sel si nécessaire), leur capacité à refroidir rapidement les produits et leur facilité d'utilisation (un simple brassage suffit à refroidir une cuve de produits de façon homogène).

Leur utilisation est également possible pour réfrigérer ou saler les produits.

Actuellement, aucun dispositif automatique, instantané et en continue ne mesure précisément et à n'importe quelle température la salinité d'une saumure. L'absence de parfaite connaissance de la concentration en NaCl de la saumure est un problème. Une concentration trop faible peut entrainer une prise en glace à la surface des serpentins ou échangeurs à plaque ce qui les isole du reste de la cuve et réduit la capacité d'échange avec le contenu de la cuve
En outre, une formation de glace dans la saumure circulant dans les systèmes d'échangeurs thermiques peut entrainer des pannes et des dommages physiques sur les équipements.

Une technique utilisée pour la mesure de concentration en NaCl de la saumure est la densimétrie (méthode du baumé). Il existe, en effet, une corrélation ente la densité d'une solution aqueuse de sel et sa concentration en sel lorsque celle-ci est « propre », c'est à dire en l'absence de matière autre. Le problème est que, à l'usage, la saumure se charge en matière organique provenant des produits et s'appauvrit en sel par absorption par les produits. Dans ce cas, la mesure de densité n'est plus corrélée à la concentration de sel. En effet, la densité sera alors dépendante de la quantité de sel en solution ainsi que des nombreuses particules en suspension.

Il est aujourd'hui très difficile :
- de mesurer une forte salinité (typiquement à 20 %) à une température négative, et/ou
- de mesurer une salinité dans une saumure souillée par de la matière organique en suspension.

La mesure selon l'état de l'art étant imprécise, les opérateurs rajoutent d'importantes quantités de sel dans la saumure, afin de la saturer et de la refroidir sans craindre la formation de cristaux de glace. Néanmoins, cette solution est loin d'être satisfaisante car elle a pour conséquence de favoriser l'absorption de sel dans les produits, et elle n'est pas suffisamment fiable et précise pour descendre à des températures inférieures à -15°C sans risquer la formation de glace.

Cette contrainte technique, à laquelle les solutions présentes sur le marché ne permettent pas d'apporter une réponse, oblige les opérateurs à renouveler très souvent leur saumure. On sait en effet, qu'avec les outils actuels, il n'est pas possible de contrôler le taux de sel par densimétrie. De plus, comme déjà évoqué, les opérateurs saturent souvent la solution pour maintenir un taux de sel élevé, au risque de faire précipiter le sel sous forme de dihydrates de sodium.

On connait aussi des mesures de concentration par réfractométrie, qui vise à déterminer la partie réelle de l'indice de réfraction d'un liquide. Ce dernier est déterminé par le comportement de la lumière à l'interface entre l'air et le liquide (réfraction et réflexion de la lumière). La connaissance de l'indice de réfraction d'une saumure « propre » permet ainsi de déterminer sa concentration en NaCl.

On connait enfin des mesures de concentration par conductivité, qui est corrélée à la salinité.

Un facteur majeur de dégradation d'une telle mesure est la polarisation.

Imposer un courant électrique à des électrodes en solution risque de provoquer une accumulation d'espèces ioniques à proximité de la surface de l'électrode et d'entraîner des réactions à la surface. Ceci va augmenter la résistance de polarisation à la surface de l'électrode, laquelle peut être considérée comme un élément parasite puisque l'on cherche à mesurer uniquement la résistance de la solution Rsol.

Le passage d'un courant électrique dans la solution peut altérer soit les électrodes (dissolution, déposition de matière, apparition d'une résistance électrique), soit la solution (dépôt de matière, diminution de sels ayant servis à la conduction).

En outre, selon les types d'électrodes utilisées (par exemple respectivement 2 pôles ou 4 pôles), la mesure peut être limitée à un domaine de concentration (respectivement faible ou haute concentration).

Enfin, les mesures de conductivité sont fortement dépendantes de la température. Au fur et à mesure que la température d'un échantillon augmente, la viscosité de l'échantillon diminue, d'où un accroissement de la mobilité des ions. Par conséquent, la conductivité observée de l'échantillon augmente aussi, même si la concentration d'ions reste constante.

On ne peut pas comparer des mesures effectuées sur un même échantillon à des températures différentes. Pour cette raison, les mesures se réfèrent à une température de référence, ce qui rend les résultats comparables. La température de référence est généralement de 25°C, parfois de 20°C.

Dans la pratique, le conductimètre mesure la conductivité à la température réelle et convertit la valeur mesurée à la température de référence souhaitée en utilisant des algorithmes de correction choisis par l'utilisateur.

La compensation en température de la conductivité à une température de référence implique que l'on connaisse typiquement un coefficient de correction. Or, celui-ci dépend de la concentration en ions. Une mesure précise de la concentration d'une solution aqueuse par la conductivité compensée en température n'est donc pas possible selon l'état de l'art.

KR 2016 0075396 décrit un dispositif de mesure de la salinité
US 7 998 513 décrit un procédé de commande et surveillance de concentration d'ascorbate.

Le but de la présente invention est de résoudre au moins un des problèmes suivants lors de la mesure de la concentration d'au moins un soluté dans une solution:
- amélioration de la précision de mesure de la concentration du soluté, et/ou
- donner la possibilité de mesurer une forte concentration en sel (typiquement à 20 %) à une température négative, et/ou
- donner la possibilité de mesurer une concentration dans une solution souillée par de la matière organique en suspension, et/ou
- éviter de gaspiller trop de sel, et/ou
- éviter de trop saler des produits contenus dans la solution, et/ou
- permettre des mesures à de très basses températures (typiquement inférieures à -15°C), et/ou
- ne pas altérer la solution, et/ou
- permettre une mesure aussi bien à faible qu'à haute concentration, et/ou
- permettre d'éviter la formation de glace et/ou d'hydrate.

### Exposé de l'invention

Cet objectif est atteint avec un procédé de mesure d'une concentration d'au moins un sel et/ou d'au moins un soluté dans une solution, comprenant les étapes suivantes :
- un stockage de données reliant la concentration Sal de la solution avec la conductivité électrique C de la solution et la température T de la solution,
- une mesure de température Tₘₑₛ de la solution,
- une mesure de conductivité électrique Cₘₑₛ de la solution,

le couple Tₘₑₛ, Cₘₑₛ de la mesure de température et de la mesure de conductivité électrique n'étant pas compris dans les données stockées reliant la concentration Sal avec la conductivité électrique C et la température T de la solution,
le procédé comprenant en outre :
   - une interpolation, en fonction des données stockées reliant la concentration Sal avec la conductivité électrique C et la température T de la solution:
      ∘ d'une loi interpolée reliant la concentration de la solution en fonction de la conductivité électrique de la solution ou inversement, pour une valeur fixe de la température de la solution égale à la température mesurée Tₘₑₛ et/ou
      ∘ d'une loi interpolée reliant la concentration de la solution en fonction de la température de la solution ou inversement, pour une valeur fixe de la conductivité électrique de la solution égale à la conductivité mesurée Cₘₑₛ
   - une détermination de la concentration Sal_{det} de la solution en fonction de cette loi interpolée et de la conductivité électrique mesurée Cₘₑₛ et/ou de la température mesurée Tₘₑₛ.

Les données stockées reliant la concentration de la solution avec la conductivité électrique et la température de la solution peuvent comprendre, pour un nombre fini de valeurs de concentration, des lois stockées chaque loi reliant température de la solution et conductivité électrique de la solution à valeur fixe de concentration de la solution.

Le nombre fini de valeurs de concentration peut comprendre au moins 3, de préférence au moins 10 valeurs de concentration distinctes.

Le nombre fini de valeurs de concentration peut comprendre au moins une valeur inférieure à un seuil eutectique et/ou peut comprendre au moins une valeur supérieure au seuil eutectique, le seuil eutectique étant de préférence égal à 23,3 % ±0,1% pour une concentration exprimée en pourcentage du poids de la solution (poids du sel dissous en gramme pour 100 grammes de solution).

Le nombre fini de valeurs de concentration sont de préférence des valeurs successives de concentration distantes de moins de 6% pour une concentration exprimée en pourcentage du poids de la solution.

Le nombre fini de valeurs de concentration peut comprendre au moins 5 valeurs de concentration distinctes supérieures à 20% et dont les valeurs successives de concentration sont distantes de moins de 1%, pour une concentration exprimée en pourcentage du poids de la solution.

Chaque loi stockée peut être une loi polynomiale.

La mesure de conductivité électrique peut comprendre une mesure de conductivité électrique par induction, comprenant un passage d'un premier courant électrique alternatif dans une première bobine conductrice induisant un deuxième courant électrique dans la solution, ce deuxième courant électrique dans la solution générant un troisième courant électrique dans une deuxième bobine.

Le procédé selon l'invention peut comprendre :
- un stockage de données sur une limite maximum de concentration de la solution en fonction de la température de la solution, et
- un calcul de la limite maximum de concentration de la solution pour la température mesurée Tₘₑₛ, à partir des données sur une limite maximum de concentration de la solution en fonction de la température.

Si la détermination de la concentration de la solution donne une valeur supérieure à la limite maximum de concentration de la solution pour la température mesurée, la détermination de la concentration de la solution peut être corrigée comme étant égale à la limite maximum de concentration de la solution pour la température mesurée.

Le procédé selon l'invention peut comprendre :
- un stockage de données sur une température T_{congel} ou T_{hydrate} de formation, dans la solution, de glace ou d'un hydrate, de préférence d'un dihydrate, sous forme solide en fonction de la concentration de la solution,
- un calcul de la température T_{congel} ou T_{hydrate} de formation, dans la solution, de glace ou d'un hydrate sous forme solide pour la concentration déterminée Sal_{det}, à partir des données sur une température T_{congel} ou T_{hydrate} de formation, dans la solution, de glace ou d'un hydrate sous forme solide en fonction de la concentration de la solution.

Le procédé selon l'invention peut comprendre :
- un calcul de la température T_{congel} de formation, dans la solution, de glace pour la concentration déterminée Sal_{det}, si la concentration déterminée Sal_{det} est inférieure ou égale à un seuil eutectique de préférence égal à 23,3 % ±0,1% pour une concentration exprimée en pourcentage du poids de la solution, ou
- un calcul de la température T_{hydrate} de formation, dans la solution, d'un hydrate sous forme solide pour la concentration déterminée Sal_{det}, si la concentration déterminée Sal_{det} est supérieure au seuil eutectique de préférence égal à 23,3 % ±0,1% pour une concentration exprimée en pourcentage du poids de la solution.

Le procédé selon l'invention peut comprendre :
- un affichage de la température T_{congel} ou T_{hydrate} calculée, et/ou
- une comparaison de la température T_{congel} ou T_{hydrate} calculée avec la température mesurée Tₘₑₛ, et, si la température T_{congel} ou T_{hydrate} calculée est supérieure ou égale à la température mesurée Tₘₑₛ, une génération d'une alarme visuelle ou sonore

En outre :
- la solution peut être ou comprendre une saumure, et/ou
- le sel ou soluté peut être ou comprendre du chlorure de sodium, et/ou
- le seuil eutectique peut être égal à 23,3 %±0,1% pour une concentration exprimée en pourcentage du poids de la solution

Suivant encore un autre aspect de l'invention, il est proposé un dispositif de mesure d'une concentration d'au moins un sel et/ou d'au moins un soluté dans solution, comprenant :
- des moyens agencés et/ou programmés pour stocker des données reliant la concentration Sal de la solution avec la conductivité électrique C de la solution et la température T de la solution,
- des moyens de mesure de température Tₘₑₛ de la solution,
- des moyens de mesure de conductivité électrique Cₘₑₛ de la solution,

le couple Tₘₑₛ, Cₘₑₛ de la mesure de température et de la mesure de conductivité électrique n'étant pas compris dans les données stockées reliant la concentration Sal avec la conductivité électrique C et la température T de la solution,
le dispositif comprenant en outre :
   - des moyens agencés et/ou programmés pour interpoler, en fonction des données stockées reliant la concentration Sal avec la conductivité électrique C et la température T de la solution:
      ∘ une loi interpolée reliant la concentration de la solution en fonction de la conductivité électrique de la solution ou inversement, pour une valeur fixe de la température de la solution égale à la température mesurée Tₘₑₛ et/ou
      ∘ une loi interpolée reliant la concentration de la solution en fonction de la température de la solution ou inversement, pour une valeur fixe de la conductivité électrique de la solution égale à la conductivité mesurée Cₘₑₛ
   - des moyens agencés et/ou programmés pour déterminer la concentration Sal_{det} de la solution en fonction de cette loi interpolée et de la conductivité électrique mesurée Cₘₑₛ et/ou de la température mesurée Tₘₑₛ.

Les données stockées reliant la concentration de la solution avec la conductivité électrique et la température de la solution peuvent comprendre, pour un nombre fini de valeurs de concentration, des lois stockées chaque loi reliant température de la solution et conductivité électrique de la solution à valeur fixe de concentration de la solution.

Le nombre fini de valeurs de concentration peut comprendre au moins 3, de préférence au moins 10 valeurs de concentration distinctes.

Le nombre fini de valeurs de concentration peut comprendre au moins une valeur inférieure à un seuil eutectique et/ou peut comprendre au moins une valeur supérieure au seuil eutectique, le seuil eutectique étant de préférence égal à 23,3 % ±0,1% pour une concentration exprimée en pourcentage du poids de la solution.

Le nombre fini de valeurs de concentration peuvent être des valeurs successives de concentration distantes de moins de 6% pour une concentration exprimée en pourcentage du poids de la solution.

Le nombre fini de valeurs de concentration peut comprendre au moins 5 valeurs de concentration distinctes supérieures à 20% et dont les valeurs successives de concentration sont distantes de moins de 1%, pour une concentration exprimée en pourcentage du poids de la solution.

Chaque loi stockée peut être une loi polynomiale.

Les moyens de mesure de conductivité électrique peuvent comprendre des moyens de mesure de conductivité électrique par induction, comprenant des moyens pour faire passer d'un premier courant électrique alternatif dans une première bobine conductrice induisant un deuxième courant électrique dans la solution, ce deuxième courant électrique dans la solution générant un troisième courant électrique dans une deuxième bobine.

Le dispositif selon l'invention peut comprendre :
- des moyens agencés et/ou programmés pour stocker des données sur une limite maximum de concentration de la solution en fonction de la température de la solution, et
- des moyens agencés et/ou programmés pour calculer la limite maximum de concentration de la solution pour la température mesurée Tₘₑₛ, à partir des données sur une limite maximum de concentration de la solution en fonction de la température.

Les moyens agencés et/ou programmés pour déterminer la concentration Sal_{det} de la solution peuvent être agencés et/ou programmés pour que, si la détermination de la concentration de la solution donne une valeur supérieure à la limite maximum de concentration de la solution pour la température mesurée, corriger la concentration de la solution comme étant égale à la limite maximum de concentration de la solution pour la température mesurée.

Le dispositif selon l'invention peut comprendre :
- des moyens agencés et/ou programmés pour stocker des données sur une température T_{congel} ou T_{hydrate} de formation, dans la solution, de glace ou d'un hydrate, de préférence d'un dihydrate, sous forme solide en fonction de la concentration de la solution,
- des moyens agencés et/ou programmés pour calculer la température T_{congel} ou T_{hydrate} de formation, dans la solution, de glace ou d'un hydrate sous forme solide pour la concentration déterminée Sal_{det}, à partir des données sur une température T_{congel} ou T_{hydrate} de formation, dans la solution, de glace ou d'un hydrate sous forme solide en fonction de la concentration de la solution.

Le dispositif selon l'invention peut comprendre :
- des moyens agencés et/ou programmés pour calculer la température T_{congel} de formation, dans la solution, de glace pour la concentration déterminée Sal_{det}, si la concentration déterminée Sal_{det} est inférieure ou égale à un seuil eutectique de préférence égal à 23,3 % ±0,1% pour une concentration exprimée en pourcentage du poids de la solution, ou
- des moyens agencés et/ou programmés pour calculer la température T_{hydrate} de formation, dans la solution, d'un hydrate sous forme solide pour la concentration déterminée Sal_{det}, si la concentration déterminée Sal_{det} est supérieure au seuil eutectique de préférence égal à 23,3 % ±0,1% pour une concentration exprimée en pourcentage du poids de la solution.

Le dispositif selon l'invention peut comprendre :
- des moyens pour afficher la température T_{congel} ou T_{hydrate} calculée, et/ou
- des moyens agencés et/ou programmés pour comparer la température T_{congel} ou T_{hydrate} calculée avec la température mesurée Tₘₑₛ, et, si la température T_{congel} ou T_{hydrate} calculée est supérieure ou égale à la température mesurée Tₘₑₛ, pour générer une alarme visuelle ou sonore.

En outre :
- la solution peut être ou comprendre une saumure, et/ou
- le sel ou soluté peut être ou comprendre du chlorure de sodium, et/ou
- le seuil eutectique peut être égal à 23,3 %±0,1% pour une concentration exprimée en pourcentage du poids de la solution

Suivant encore un autre aspect de l'invention, il est proposé un bateau ou navire ou installation à terre ou usine équipé d'un dispositif selon l'invention ou mettant en œuvre le procédé selon l'invention.

### Description des figures et modes de réalisation

D'autres avantages et particularités de l'invention apparaîtront à la lecture de la description détaillée de mises en œuvre et de modes de réalisation nullement limitatifs, et des dessins annexés suivants :
**[****Fig. 1****]** la figure 1 illustre un premier mode de réalisation de dispositif 1 selon l'invention, qui est le mode de réalisation préféré de l'invention,
**[****Fig. 2****]** la figure 2 est un organigramme illustrant différentes étapes d'un premier mode de réalisation de procédé selon l'invention mis en œuvre par le premier mode de réalisation de dispositif 1 selon l'invention,
**[****Fig. 3****]** la figure 3 illustre des données stockées 7 reliant la concentration Sal en fonction d'un couple conductivité électrique C et température T de la solution,
**[****Fig. 4****]** la figure 4 illustre un diagramme de phase de saumure, et l'effet d'un refroidissement sur la saumure pour une « faible » concentration en sel,
**[****Fig. 5****]** la figure 5 illustre un diagramme de phase de saumure, et l'effet d'un refroidissement sur la saumure pour une « forte » concentration en sel,,
**[****Fig. 6****]** la figure 6 illustre un diagramme de phase de saumure,
**[****Fig. 7****]** la figure 7 illustre une interpolation pour une détermination d'une concentration dans le premier mode de réalisation du procédé selon l'invention, et
**[****Fig. 8****]** la figure 8 illustre une détermination d'une concentration dans le premier mode de réalisation du procédé selon l'invention, basée sur l'interpolation de la figure 7.

Ces modes de réalisation étant nullement limitatifs, on pourra notamment considérer des variantes de l'invention ne comprenant qu'une sélection de caractéristiques décrites ou illustrées par la suite isolées des autres caractéristiques décrites ou illustrées (même si cette sélection est isolée au sein d'une phrase comprenant ces autres caractéristiques), si cette sélection de caractéristiques est suffisante pour conférer un avantage technique ou pour différencier l'invention par rapport à l'état de la technique antérieure. Cette sélection comprend au moins une caractéristique de préférence fonctionnelle sans détails structurels, et/ou avec seulement une partie des détails structurels si cette partie uniquement est suffisante pour conférer un avantage technique ou à différencier l'invention par rapport à l'état de la technique antérieure.

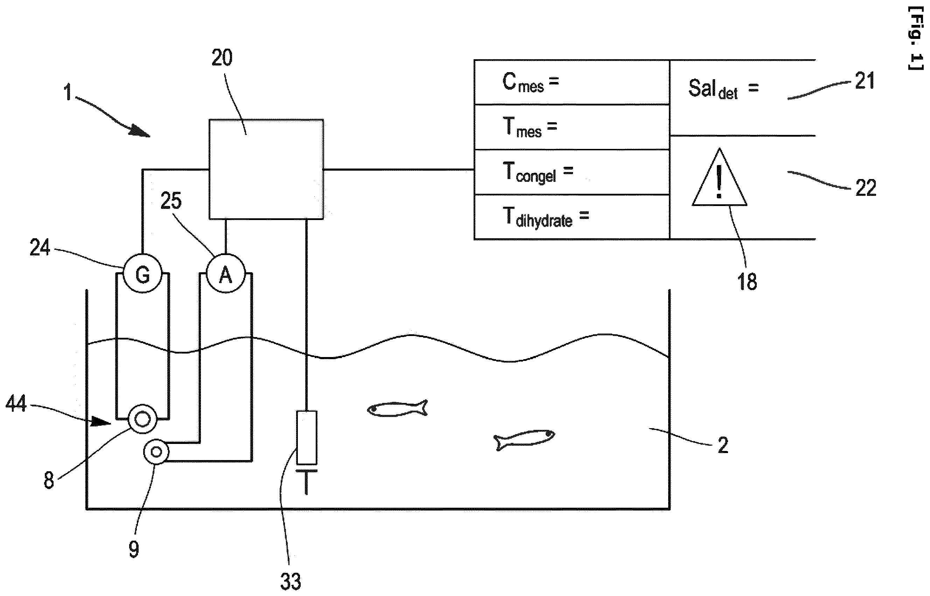

On va tout d'abord décrire, en référence aux figures 1 à 8, un premier mode de réalisation de dispositif 1 de mesure d'une concentration d'au moins un sel et/ou d'au moins un soluté dans solution 2, mettant en œuvre un premier mode de réalisation de procédé selon l'invention.

Dans la présente description, on entendra toujours par « concentration » la concentration d'au moins un sel dissous et/ou d'au moins un soluté dans solution (cette solution étant la somme du solvant et de l'ensemble des solutés).

La solution 2 est de préférence une saumure.

Une saumure est une solution aqueuse d'un sel (mélange d'eau, le solvant, et de sel, généralement du chlorure de sodium NaCl, le soluté). Une solution est dite saturée lorsque le soluté introduit ne peut plus se dissoudre (et forme un précipité).

En chimie, un sel est un composé ionique composé de cations et d'anions formant un produit neutre et sans charge nette. Ces ions peuvent être aussi bien minéraux (chlorure Cl⁻) qu'organiques (acétate CH3-COO⁻) et monoatomiques (fluorure F⁻) aussi bien que polyatomiques SO₄²⁻).

L'au moins un soluté est de préférence :
- un sel dissous dans la solution, et/ou
- au moins un sel dissous dans la solution, et/ou
- l'ensemble des sels dissous dans la solution, et/ou
- du chlorure de sodium dissous dans la solution.

On détaillera par la suite plus en détail l'invention dans un exemple où la concentration correspond à une concentration de NaCl dans de l'eau. En effet, une étude réalisée en laboratoire sur des saumures prélevées sur des navires a révélé que plus de 97% des sels dissous était du NaCl. L'invention reste cependant généralisable à d'autre(s) sel(s) ou soluté(s).

Par concentration, on entend de préférence une salinité de la solution, i.e. la concentration d'un sel (de préférence du Chlorure de Sodium NaCl) ou d'au moins un des sels (de préférence du Chlorure de Sodium NaCl) ou de tous les sels présent(s) dans la solution.

Cette concentration peut être exprimée comme n'importe quel rapport d'unité de mesure d'une quantité (par exemple mole, volume, masse) de sel dissous ou soluté par unité de mesure d'une quantité (par exemple mole, volume, masse) de solution. Si ces deux quantités sont les mêmes (par exemple le gramme de sel dissous par gramme de solution), cette concentration peut être par exemple exprimée en pourcentage.

Cette concentration peut donc être par exemple exprimée :
a) en masse de sel dissous par masse de solution (par exemple g/g ou %), et/ou
b) en molécules de sel dissoutes par masse ou volume de solution (par exemple mol/g ou mol/L ou mol/dm3), et/ou
c) en masse de sel dissous par volume de solution (par exemple g/L ou g/dm3), et/ou
d) ou n'importe quelle donnée proportionnelle ou liée par une fonction bijective à l'une de ces données précédentes a) à c) ci-dessus (de préférence à température donnée), par exemple en unité Bé (degré Baumé) qui correspond à une habitude de l'homme du métier pour exprimer des concentrations de sel dans une saumure.

Par défaut, toute concentration dont la valeur est exprimée en pourcentage dans la présente description correspond à un pourcentage du poids de la solution, i.e. en pourcentage de gramme de sel (NaCl) dissous par gramme de solution (H₂0+NaCl dissous)
Un « hydrate » est un composé ionique qui contient des molécules d'eau dans son réseau cristallin, un préfixe (comme di-, tri-, etc...) indiquant le nombre de molécules d'eau associées aux ions. Lorsque l'on refroidit une saumure de NaCl de taux massique supérieur à 23,3 %, une partie du sel dissous va recristalliser sous forme de dihydrate (dihydrate de sodium NaCl-2H₂O dite phase hydrohalite). L'hydrohalite formée à partir de solutions de sel et de sel solide a un aspect et une structure proche de ceux de la glace.

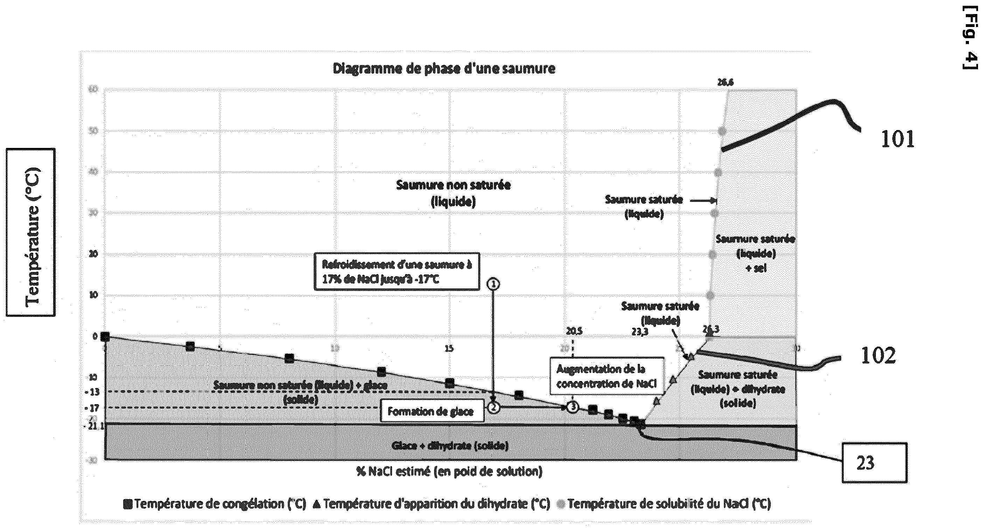

Un diagramme de phases de saumure de NaCl dissous dans de l'eau est représenté sur chacune des figures 4, 5 et 6. On peut distinguer plusieurs états, dont :
- saumure non saturée en sel: du sel peut encore y être dissous dans de l'eau liquide.
- mélange de saumure non saturée et de glace : cet état est obtenu lorsque l'on refroidit une saumure non saturée (de concentration en NaCl inférieure à 23,3%) à une température inférieure à sa température de congélation. 23,3% correspond au point eutectique (référencé 23 dans le diagramme). A 23,3% le mélange eau - NaCl est dit eutectique car il se comporte comme un corps pur du point de vue de la congélation : on passe directement d'un état liquide à un état solide à la température de congélation, contrairement aux autres concentrations ou le changement de température conduit à un mélange liquide + solide (ici de la glace H₂O pur).
- saumure saturée : la saumure contient la quantité maximale de sel dissous possible à la température considérée. Cette quantité de sel maximale que l'on peut dissoudre dépend de la température. Lorsque la saumure est saturée, un rajout de sel se traduit par une précipitation de ce dernier au fond du récipient ou une formation de dihydrate (glace salée). La saumure saturée correspond à un état dit « eutectique ».
- saumure saturée + dihydrate: cet état est obtenu lorsque l'on refroidit une saumure saturée ou non saturée de concentration supérieure à 23,3%. Lorsque la température de la saumure diminue, la solubilité du sel diminue également. L'excès de sel conduit alors à la formation de glace salée (dihydrate). Lorsque la température de formation du dihydrate est atteinte, l'excès de sel disparait de la saumure et la concentration de cette dernière diminue jusqu'à atteindre sa valeur de saturation.

Par "eutectique », on entend un mélange de deux ou plusieurs corps purs qui fond et se solidifie à température constante de manière uniforme, contrairement aux mélanges habituels où le changement de température conduit à une variation de la proportion de solide par rapport à celle de liquide. Il se conduit en fait comme un corps pur du point de vue de la solidification et de la fusion.

Par « point eutectique » référencé 23 sur les figures 4 à 6, on entend le point du diagramme de phase (température en fonction de la concentration) pour lequel la solution ou le mélange (soluté + solvant) est à sa température minimale en phase liquide. Cette température est propre à chaque mélange.

Par « seuil eutectique » de la concentration, on entend la valeur de la concentration en sel ou soluté au point eutectique.

Pour une saumure de chlorure de sodium (mélange eau + Chlorure de sodium), le seuil eutectique est de 23,3% (±0,1%) gramme de NaCl dissous par gramme de solution NaCl+eau.

Le titre massique maximal pour une saumure de chlorure de sodium à 0° est de 26,3 %.

Comme illustré en Figure 4, en cas de refroidissement d'une saumure à concentration en NaCL inférieure à 23,3 %, une partie de l'eau va cristalliser sous forme de glace, ce qui va entrainer une augmentation de la concentration en NaCl. Dans l'exemple de la Figure 4, une saumure de concentration de NaCl à 17% est descendue en froid jusqu'à atteindre une température de -17°C. La température de congélation d'une telle saumure étant -13°C, de la glace d'eau pure va se former. La glace en se formant va « capturer » une partie de l'eau présent dans la saumure. Cette dernière va donc voir sa concentration augmenter jusqu'à atteindre une valeur lui permettant de rester à l'état liquide. Cette valeur est 20,5% car une telle concentration correspond à une température de congélation de -17°C. Si la température continue de baisser, la concentration de la saumure va continuer d'augmenter jusqu'à atteindre 23,3% à -21,1°C. En dessous de cette température la saumure disparait et laisse place à un mélange solide glace + glace salée (dihydrate).

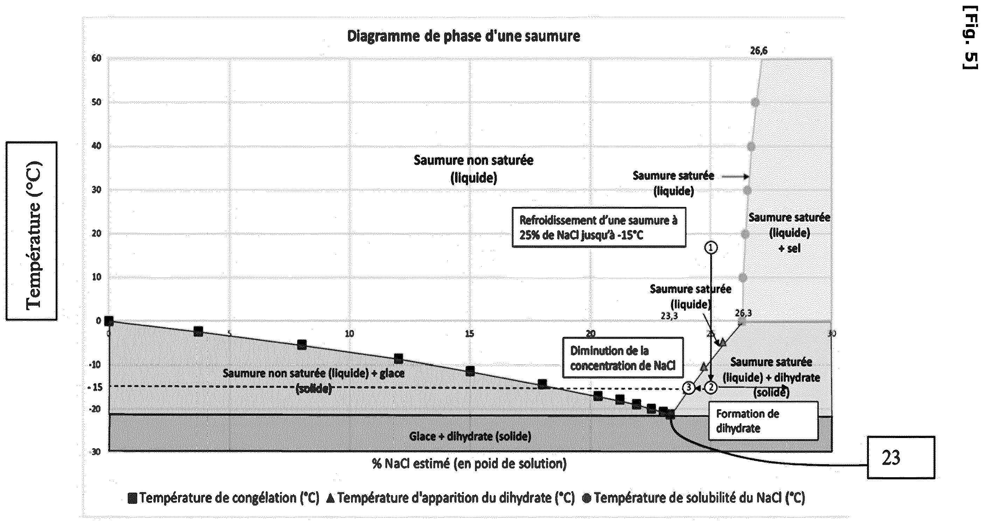

Comme illustré en Figure 5, en cas de refroidissement d'une saumure à forte concentration en NaCL (supérieure à 23,3 %) telle une saumure saturée, une partie du sel dissous va recristalliser sous forme de dihydrate, ce qui va entrainer une diminution de la concentration en NaCl.

Selon ce mode de réalisation de l'invention, chacun des moyens décrits dans la présente description:
- pour stocker, et/ou
- pour interpoler, et/ou
- pour déterminer, et/ou
- pour calculer, et/ou
- pour comparer, et/ou
- de manière générale mentionné(s) comme pouvant être programmé pour accomplir une fonction,
comprend des moyens techniques, et comprend de préférence au moins un ordinateur, une unité centrale ou de calcul, un circuit électronique analogique (de préférence dédié), un circuit électronique numérique (de préférence dédié), et/ou un microprocesseur (de préférence dédié), et/ou des moyens logiciels.

Selon ce mode de réalisation, l'ensemble des moyens décrits dans la présente description:
- pour stocker (des données), et/ou
- pour interpoler, et/ou
- pour déterminer, et/ou
- pour calculer, et/ou
- pour comparer, et/ou
- **de manière** générale programmé pour accomplir n'importe quelle fonction,
consistent en une même unité de calcul 20 comprenant de préférence au moins un ordinateur, une unité centrale ou de calcul, un circuit électronique analogique (de préférence dédié) et/ou un circuit électronique numérique (de préférence dédié), et/ou un microprocesseur (de préférence dédié), et/ou des moyens logiciels.

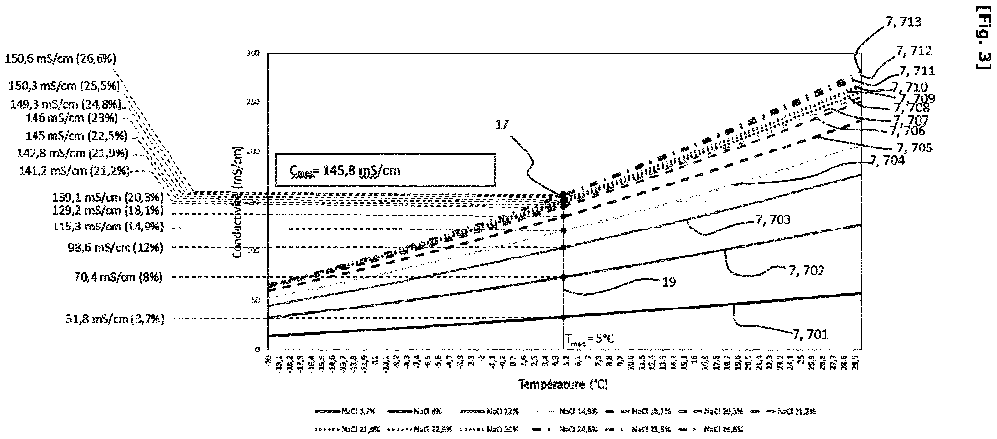

En référence à la Figure 1, le dispositif 1 comprend :
- des moyens 20 agencés et/ou programmés pour stocker des données 7 (illustrées sur la figure 3) reliant la concentration en sel dissous (notée par la suite « Sal » ou aussi notée « %NaCl » dans l'exemple d'une solution de NaCl) de la solution 2 en fonction d'un couple constitué de la conductivité électrique C de la solution 2 et de la température T (comprise typiquement au moins entre -20 et 20°C pour ces données 7, i.e. avec au moins une valeur inférieure ou égale à -20°c et au moins une valeur supérieure ou égale à 20°C ou même 30°C) de la solution 2,
- des moyens 33 de mesure (étape 3) de température Tₘₑₛ de la solution 2, comprenant typiquement une sonde à résistance par exemple de référence Endress+Hauser Easytemp TMR31,
- des moyens 44 de mesure (étape 4) de conductivité électrique Cₘₑₛ de la solution 2, par exemple de référence Endress+Hauser Indumax CLS50D,

Le dispositif 1 comprend en outre :
- des moyens 20 agencés et/ou programmés pour interpoler (étape 5), en fonction des données stockées 7 reliant la concentration Sal en fonction du couple conductivité électrique C et température T de la solution 2:
   ∘ une loi appelée « loi interpolée » 19 reliant la concentration Sal de la solution 2 en fonction de la conductivité électrique C de la solution 2 ou inversement, pour une valeur fixe de la température de la solution égale à la température mesurée Tₘₑₛ (nous décrirons plus en détail cette première variante par la suite), et/ou
   ∘ une loi appelée « loi interpolée » 19 reliant la concentration Sal de la solution en fonction de la température T de la solution ou inversement, pour une valeur fixe de la conductivité électrique de la solution égale à la conductivité mesurée Cₘₑₛ (nous ne décrirons pas plus en détail cette deuxième variante par la suite, son principe étant le même que la première variante)
- des moyens 20 agencés et/ou programmés pour déterminer (étape 6) la concentration (notée par la suite « Sal_{det}» pour sa valeur déterminée) de la solution 2 en fonction de cette loi interpolée 19 et de la conductivité électrique mesurée Cₘₑₛ (en particulier pour la première variante) et/ou de la température mesurée Tₘₑₛ (en particulier pour la deuxième variante).

Le dispositif 1 est agencé (et en particulier les moyens 20 pour déterminer et/ou pour interpoler sont agencés) pour fonctionner même si le couple 17 Tₘₑₛ, Cₘₑₛ de la mesure de température et de la mesure de conductivité électrique n'est pas compris dans les données stockées 7 reliant la concentration Sal en fonction du couple conductivité électrique C et température T de la solution 2.

Comme illustré en Figure 3, les données stockées 7 reliant la concentration Sal de la solution 2 avec la conductivité électrique C et la température T de la solution 2 comprennent, pour un nombre fini de valeurs de concentration Sal, des lois 7, 701-713 stockées, chaque loi stockée 701-713 ou fonction stockée 701-713 reliant température T de la solution et conductivité électrique C de la solution 2 à valeur fixe de concentration Sal de la solution 2 (dans une alternative, les données 7 peuvent par exemple comprendre un tableau de valeurs à trois entrées Sal, T et C).

Ces *« données stockées 7 reliant la concentration Sal de la solution 2 avec la conductivité électrique C et la température T de la solution 2* » peuvent donc comprendre au moins une loi, au moins une fonction, au moins un tableau de valeurs, au moins une base de données, etc. peu importe la forme de ces données.

Le nombre fini de valeurs de concentration Sal comprend au moins 3, de préférence au moins 10 valeurs de concentration Sal distinctes.

Le nombre fini de valeurs de concentration Sal comprend au moins une valeur inférieure à 5 % ou même à 4% (Sal étant égal à 3,5% pour de l'eau de mer) et/ou comprend au moins une valeur supérieure à 20% ou même à 25%, pour une concentration exprimée en pourcentage du poids de la solution 2.

Le nombre fini de valeurs de concentration Sal comprend au moins une valeur inférieure au seuil eutectique et/ou comprend au moins une valeur supérieure au seuil eutectique, le seuil eutectique étant de préférence égal à 23,3 % pour une concentration exprimée en pourcentage du poids de la solution 2.

Le nombre fini de valeurs de concentration Sal sont des valeurs successives de concentration distantes de moins de 6% pour une concentration exprimée en pourcentage du poids de la solution 2.

Le nombre fini de valeurs de concentration Sal comprend au moins 5 valeurs de concentration distinctes supérieures à 20% et dont les valeurs successives de concentration sont distantes de moins de 1%, pour une concentration exprimée en pourcentage du poids de la solution 2.

Dans le cas particulier de la figure 3, la concentration Sal est une concentration de NaCl dissous dans de l'eau.

Dans le cas particulier de la figure 3, le nombre fini de valeurs de concentration Sal des données 7 comprend :
- 26,6% de gramme de NaCl dissous par gramme de solution NaCl+eau,
- 25,5% de gramme de NaCl dissous par gramme de solution NaCl+eau,
- 24,8% de gramme de NaCl dissous par gramme de solution NaCl+eau,
- 23% de gramme de NaCl dissous par gramme de solution NaCl+eau,
- 22,5% de gramme de NaCl dissous par gramme de solution NaCl+eau,
- 21,9% de gramme de NaCl dissous par gramme de solution NaCl+eau,
- 21,2% de gramme de NaCl dissous par gramme de solution NaCl+eau,
- 20,3% de gramme de NaCl dissous par gramme de solution NaCl+eau,
- 18,1% de gramme de NaCl dissous par gramme de solution NaCl+eau,
- 14,9% de gramme de NaCl dissous par gramme de solution NaCl+eau,
- 12% de gramme de NaCl dissous par gramme de solution NaCl+eau,
- 8% de gramme de NaCl dissous par gramme de solution NaCl+eau,
- 3,7% de gramme de NaCl dissous par gramme de solution NaCl+eau.

Chaque loi stockée 7 est une loi polynomiale, de préférence de degré au moins 3 (de degré 3 dans le cas illustré sur la figure 3).

Les moyens de mesure de conductivité électrique 44 comprennent des moyens de mesure de conductivité électrique par induction, comprenant :
- une première bobine conductrice 8 (de préférence toroïdale),
- une deuxième bobine conductrice 9 (de préférence toroïdale) appelée « pick-up tore »,
- des moyens 24 (comprenant typiquement un générateur de courant électrique alternatif) agencés pour faire passer un premier courant électrique alternatif dans la première bobine 8 de manière à induire un deuxième courant électrique dans la solution 2, la deuxième bobine 9 étant agencée pour que ce deuxième courant électrique dans la solution génère un troisième courant électrique dans la deuxième bobine 9,
- des moyens 25 (comprenant typiquement un ampèremètre et/ou une détection synchrone reliée aux moyens 24) agencés pour mesurer le troisième courant électrique circulant dans la deuxième bobine 9.

Le dispositif 1 comprend des moyens 20 agencés et/ou programmés pour calculer la conductivité C en fonction de ce troisième courant électrique.

La bobine 8 est entourée d'un matériau de protection agencé pour éviter tout contact direct de la bobine 8 avec la solution 2.

La bobine 9 est entouré d'un matériau de protection agencé pour éviter tout contact électrique direct de la bobine 9 avec la solution 2.

Par matériau de protection, on entend toute matière (par exemple plastique ou polymère) de forte résistance chimique et de résistance élevée à la température et aux contraintes mécaniques.

Le dispositif 1 comprend en outre:
- des moyens 20 agencés et/ou programmés pour stocker des données sur une limite maximum de concentration %Max de la solution 2 en fonction de la température T de la solution 2 ; ces données sont typiquement mémorisées sous la forme d'un tableau de valeurs ou d'une fonction reliant la limite maximum de concentration de la solution 2 en fonction de la température T de la solution, et correspondent à la ligne de jonction (illustrée sur les figures 4 à 6) entre d'une part la phase saumure non saturée et d'autre part la phase saumure saturée ou la phase saumure saturée + dihydrate ; et
- des moyens 20 agencés et/ou programmés pour calculer ou déterminer (étape 10) la limite maximum de concentration de la solution %Max pour la température mesurée Tₘₑₛ, à partir des données sur une limite maximum de concentration de la solution en fonction de la température.

Les moyens 20 agencés et/ou programmés pour interpoler (étape 5) (en fonction des données stockées 7 reliant la concentration Sal avec la conductivité électrique C et la température T de la solution 2) la loi ou formule ou relation 19 sont agencés pour, lors de cette interpolation, éliminer des données 7 utilisées pour l'interpolation les données correspondant à une concentration Sal supérieure à la concentration maximum %Max à la température Tₘₑₛ.

Les moyens 20 agencés et/ou programmés pour déterminer (étape 6) la concentration Sal_{det} de la solution 2 sont agencés et/ou programmés pour que, si la détermination de la concentration de la solution donne une valeur supérieure à la limite maximum de concentration %Max de la solution 2 pour la température mesurée Tₘₑₛ, corriger (étape 11) la concentration de la solution déterminée Sal_{det} comme étant égale à la limite maximum de concentration %Max de la solution 2 pour la température mesurée Tₘₑₛ.

Le dispositif 1 comprend :
- des moyens 20 agencés et/ou programmés pour stocker des données sur une température T_{congel} ou T_{hydrate} de formation, dans la solution 2, de glace ou d'un hydrate, de préférence d'un dihydrate, sous forme solide en fonction de la concentration Sal de la solution 2,
- des moyens 20 agencés et/ou programmés pour calculer la température T_{congel} ou T_{hydrate} de formation, dans la solution (2), de glace ou d'un hydrate sous forme solide pour la concentration déterminée Sal_{det}, à partir des données sur une température T_{congel} ou T_{hydrate} de formation, dans la solution, de glace ou d'un hydrate sous forme solide en fonction de la concentration Sal de la solution 2.

Le dispositif 1 comprend :
- des moyens 20 agencés et/ou programmés pour calculer (étape 12) la température T_{congel} de formation, dans la solution, de glace pour la concentration déterminée Sal_{det}, si la concentration déterminée Sal_{det} est inférieure ou égale au seuil eutectique de préférence égal à 23,3 % (±0,1%) pour une concentration exprimée en pourcentage du poids de la solution, ou
- des moyens 20 agencés et/ou programmés pour calculer (étape 13) la température T_{hydrate} de formation, dans la solution, d'un hydrate sous forme solide pour la concentration déterminée Sal_{det}, si la concentration déterminée Sal_{det} est supérieure au seuil eutectique de préférence égal à 23,3 % (±0,1%) pour une concentration exprimée en pourcentage du poids de la solution.

Le dispositif 1 comprend :
- des moyens 21 (tel un écran) pour afficher (étape 14) la température T_{congel} ou T_{hydrate} calculée, et/ou
- des moyens 20 agencés et/ou programmés pour comparer (étape 15) la température T_{congel} ou T_{hydrate} calculée avec la température mesurée Tₘₑₛ, et, si la température T_{congel} ou T_{hydrate} calculée est supérieure ou égale à la température mesurée Tₘₑₛ, des moyens 22 (comprenant un écran, un haut parleur, etc.) pour générer (étape 16) une alarme 18 visuelle ou sonore.

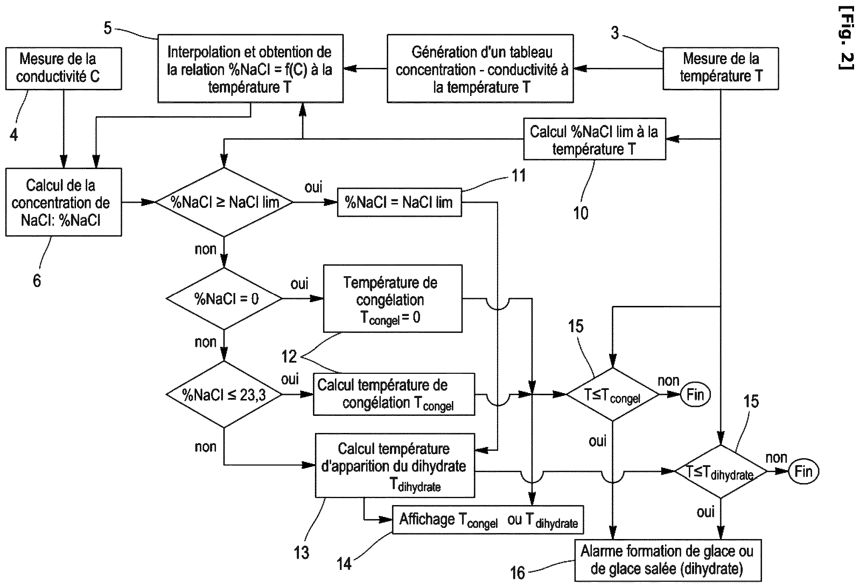

En référence à la Figure 2, le premier mode de réalisation de procédé selon l'invention de mesure d'une concentration Sal d'au moins un sel et/ou d'au moins un soluté dans la solution 2, comprend les étapes suivantes.

Tout d'abord, ce procédé comprend un stockage des données 7 reliant la concentration Sal de la solution en fonction d'un couple constitué de la conductivité électrique C et de la température T de la solution.

Ces données 7 sont obtenues de la manière suivante (et peuvent être réalisées et stockées dans le dispositif 1 pour n'importe quel(s) sel(s) dissous dans l'eau ou n'importe quel couple soluté/solvant, selon la variante considérée) : des saumures de concentrations entre 3,5% de NaCl et 26,6% de NaCl ont été fabriquées (on calcule précisément la quantité de sel à introduire dans un certain volume d'eau de mer pour obtenir précisément la concentration attendue, en tenant compte de la teneur initiale de l'eau de mer en sel ainsi que l'augmentation de son volume une fois le sel dissous ; des mesures au réfractomètre et au densimètre ont également permis de valider la concentration (la saumure n'étant pas chargée en matière organique en suspension susceptible de fausser la mesure)) puis les saumures ont été refroidies de la température ambiante jusqu'à -20°C à l'aide d'un cryostat. Ce dernier refroidi un bain de liquide caloporteur qui, à son tour, refroidi le récipient de saumure. La conductivité et la température de la saumure sont mesurées le long de la descente en froid. Enfin, la formation de glace étant un phénomène exothermique, un palier de température se forme lors de l'apparition de glace. L'observation de palier permet de déterminer la température de congélation T_{congel} associée à chaque concentration de NaCl qui sont stockées dans l'unité 20. Ainsi, la mesure du point de congélation de chaque saumure de concentration inférieure à 23,3% est réalisée. Il suffit pour cela de descendre la température du liquide caloporteur jusqu'à observer une remonté de température de la saumure. Ceci est dû au phénomène de cristallisation qui est métastable. Cela signifie que la saumure peut rester en phase liquide alors que sa température est plus basse que son point de congélation. Au moment où la glace se forme, la température de la saumure remonte jusqu'à sa température de congélation car la réaction chimique de cristallisation exothermique compense le refroidissement par le liquide caloporteur. Au cours de la descente en froid la conductivité de la saumure a été mesurée. Une interpolation polynomiale de degré 3 a permis d'obtenir une relation entre conductivité et température pour l'ensemble des concentrations et générer ainsi les données 7. Ainsi, comme déjà expliqué en description du dispositif 1, les données stockées 7 reliant la concentration de la solution en fonction d'un couple constitué de la conductivité électrique et de la température de la solution comprennent, pour un nombre fini de valeurs de concentration Sal, des lois 7, 701-713 stockées chaque loi respectivement 701 à 713 reliant température T de la solution 2 et conductivité électrique C de la solution 2 à valeur fixe de concentration Sal de la solution 2 par exemple respectivement 3,7%, 8%, 12%, 14,9%, 18,1%, 20,3%, 21,2%, 21,9%, 22,5%, 23%, 24,8%, 25,5% et 26,6% du poids de la solution NaCl+eau. Chaque loi stockée 7 respectivement 701 à 713 est une loi polynomiale de degré au moins 3. Chaque courbe ou loi 701 à 713 de conductivité C en fonction de la température T est donc associée à une concentration Sal et :
- à une température de congélation T_{congel} pour les saumures de concentration inférieure au seuil eutectique (23,3%), ou
- à une température de formation d'un hydrate T_{hydrate} pour les saumures de concentration supérieure au seuil eutectique (23,3%).

Ensuite, interviennent les mesures sur l'échantillon de solution 2 que l'on souhaite réellement tester.

Le procédé comprend une mesure 3, par les moyens 33, de température Tₘₑₛ de la solution 2.

Le procédé comprend une mesure 4, par les moyens 44, de conductivité électrique Cₘₑₛ de la solution 2. La mesure de conductivité électrique 4 comprend une mesure de conductivité électrique par induction, comprenant :
- un passage d'un premier courant électrique alternatif dans la première bobine conductrice 8 induisant un deuxième courant électrique dans la solution 2, ce deuxième courant électrique dans la solution générant un troisième courant électrique dans la deuxième bobine 9,
- une mesure, par les moyens 25, du troisième courant électrique circulant dans la deuxième bobine 9,
- une détermination de Cₘₑₛ par l'unité 20 à partir de la valeur mesurée de ce troisième courant.

Typiquement, le couple 17 Tₘₑₛ, Cₘₑₛ de la mesure de température et de la mesure de conductivité électrique n'est pas compris dans les données stockées 7 reliant la concentration Sal en fonction d'un couple conductivité électrique C et température T de la solution.

Ainsi, le procédé comprend une vérification (par les moyens 20 qui sont agencés et/ou programmés pour une telle étape de vérification), de si le couple 17 Tₘₑₛ, Cₘₑₛ de la mesure de température et de la mesure de conductivité électrique n'est pas compris dans les données stockées 7 reliant la concentration Sal en fonction d'un couple conductivité électrique C et température T de la solution. Ensuite, si effectivement le couple 17 Tₘₑₛ, Cₘₑₛ de la mesure de température et de la mesure de conductivité électrique n'est pas compris dans les données stockées 7 reliant la concentration Sal en fonction d'un couple conductivité électrique C et température T de la solution, le procédé comprend l'interpolation décrite ci-après (dans le cas contraire, la détermination de Sal est triviale car déterminée comme faisant partie des données déjà stockées).

Le procédé comprend donc en outre une interpolation 5 (par l'unité 20), en fonction des données stockées 7 reliant la concentration Sal en fonction d'un couple conductivité électrique C et température T de la solution:
∘ de la loi interpolée 19 reliant la concentration Sal de la solution 2 en fonction de la conductivité électrique C de la solution 2 ou reliant la conductivité électrique C de la solution 2 en fonction de la concentration Sal de la solution 2, pour une valeur fixe de la température de la solution égale à la température mesurée Tₘₑₛ et/ou
∘ de la loi interpolée 19 reliant la concentration Sal de la solution 2 en fonction de la température T de la solution 2 ou reliant la température T de la solution 2 en fonction de la concentration Sal de la solution 2 , pour une valeur fixe de la conductivité électrique de la solution égale à la conductivité mesurée Cₘₑₛ.

Une fois cette loi 19 déterminée, elle est utilisée afin de déterminer la concentration en NaCl Sal_{det} puis la température T_{congel} ou T_{hydrate}. Le procédé comprend donc une détermination 6 (par l'unité 20) de la concentration Sal_{det} de la solution 2 en fonction de cette loi interpolée et de la conductivité électrique mesurée Cₘₑₛ et de la température mesurée Tₘₑₛ.

Pour l'interpolation de la loi 19, pour chacune des concentrations qui ont servi à réaliser les données stockées 7 (3,7%, 8%, ... , 26.6%), on associe une valeur de conductivité C en connaissant la valeur de température Tₘₑₛ de la saumure. Nous obtenons donc un certain nombre de points qui peuvent apparaitre dans un graphe avec en abscisse la conductivité C et en ordonnée la concentration Sal (pour ces valeurs 3,7%, 8%, ... , 26.6%) à la température mesurée Tₘₑₛ. Une interpolation type polynomiale ou Spline (de premier ordre) est ensuite réalisée sur ces points afin d'obtenir une loi mathématique qui lie la conductivité C à la concentration Sal à la température connue Tₘₑₛ. L'interpolation 5, en fonction des données stockées 7, de la loi interpolée 19, comprend une élimination des données 7 utilisées pour l'interpolation correspondant à une concentration Sal supérieure à la concentration maximum %Max à la température Tₘₑₛ. (Dans une alternative, pour l'interpolation de la loi 19, pour chacune des concentrations qui ont servi à réaliser les données stockées 7 (3,7%, 8%, ... , 26.6%) sauf les concentrations supérieures à la concentration maximum %Max à la température Tₘₑₛ, on associe une valeur de conductivité C en connaissant la valeur de température Tₘₑₛ de la saumure. Nous obtenons donc un certain nombre de points qui peuvent apparaitre dans un graphe avec en abscisse la conductivité C et en ordonnée la concentration Sal (pour ces valeurs 3,7%, 8%, ... , 26.6%) à la température mesurée Tₘₑₛ. Une interpolation type polynomiale ou Spline (de premier ordre) est ensuite réalisée sur ces points afin d'obtenir une loi mathématique qui lie la conductivité C à la concentration Sal à la température connue Tₘₑₛ.)

En effet, le procédé comprend en outre un stockage des données sur une limite maximum de concentration %Max (aussi notée « %NaCl lim » ou « %NaCl max »dans l'exemple d'une solution de NaCl) de la solution 2 en fonction de la température T de la solution 2; en effet, il existe une concentration maximale de sel dans la saumure, celle-ci dépend de la température T. Par exemple (voir Figure 5), lorsque la température est de 25°C, la concentration maximale de sel en solution est 26,5% (un rajout de sel ne se diluera pas mais précipitera). Lorsque la température est -15°C, la concentration maximale est 24% (un rajout de sel ne se diluera pas mais formera un dihydrate avec les molécules d'eau). L'unité 20 détermine la concentration maximale et donc les lois 7 retenues lors de l'interpolation 5.

Le procédé comprend donc un calcul ou détermination 10 (par l'unité 20) de la limite maximum de concentration %Max de la solution 2 pour la température mesurée Tₘₑₛ, à partir des données sur une limite maximum de concentration de la solution en fonction de la température. Pour cela :
- soit une fois la température Tₘₑₛ mesurée, une simple interpolation du couple (Température, %Max) permet de déterminer la concentration de NaCl maximum %Max à la température Tₘₑₛ,
- soit la détermination de la limite maximum de concentration est réalisée à partir de 2 formules mathématiques stockées:
   ∘ une formule correspondant à la précipitation du sel (lorsque la température est positive) et correspondant à la ligne ou courbe 101 de la figure 4, i.e. la ligne 101 de saturation sur le diagramme de phase de la saumure entre la saumure non saturée liquide et la saumure saturée liquide + sel
   ∘ une autre formule correspondant à la formation de dihydrate (lorsque la température est négative) et correspondant à la ligne la courbe 102 de la figure 4, i.e. la ligne 102 de saturation sur le diagramme de phase de la saumure entre la saumure non saturée liquide et la saumure saturée liquide + dihydrate (solide)
Chacune de ces formules (stockées dans l'unité 20 et utilisée par l'unité 20) relie une concentration maximum %Max à une température Tmes, et permet donc simplement de déterminer %Max à partir de Tmes.

Si la détermination de la concentration de la solution donne une valeur supérieure à la limite maximum de concentration %Max de la solution 2 pour la température mesurée Tₘₑₛ (Sal_{det}>%Max(Tₘₑₛ)), la détermination de la concentration de la solution 2 est corrigée 11 (par l'unité 20) comme étant égale à la limite maximum de concentration de la solution pour la température mesurée (Sal_{det}=%Max(Tₘₑₛ)).

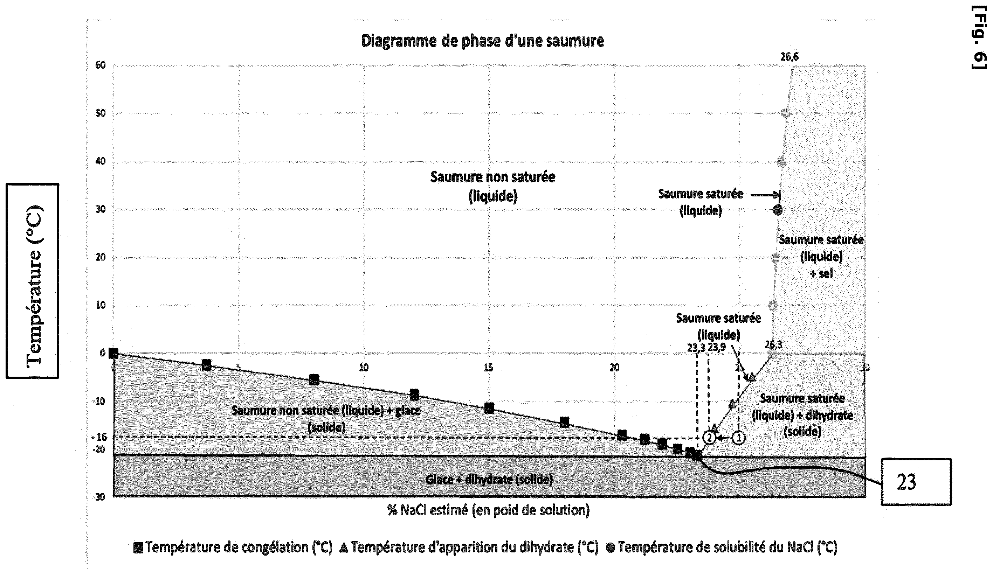

Par exemple, en référence aux figures 2 et 6, si l'étape 6 indique une valeur de Sal_{det}=25% à la température de Tₘₑₛ=-16°C, cela voudrait dire que l'on mesure une concentration dans le mélange saumure + dihydrate. Cela n'est pas possible car la sonde mesure uniquement la conductivité de la saumure liquide, qui à cette température, ne peut avoir une concentration de NaCl supérieure à %Max=23,9%. L'invention corrige donc, à l'étape 11, la valeur de concentration à Sal_{det}=23,9%.

Le procédé comprend en outre :
- un stockage des données sur la température T_{congel} ou T_{hydrate} de formation, dans la solution 2, de glace ou d'un hydrate, de préférence d'un dihydrate, sous forme solide en fonction de la concentration Sal de la solution 2; ces données sont typiquement mémorisées sous la forme d'un tableau de valeurs ou d'une fonction reliant la température T_{congel} ou T_{hydrate} de formation, dans la solution, de glace ou d'un hydrate, de préférence d'un dihydrate, en fonction de la concentration Sal de la solution 2, et correspondent à la ligne de jonction (illustrée sur les figures 4 à 6) entre d'une part la phase saumure non saturée ou la phase saumure saturée et d'autre part la phase saumure non saturée + glace ou la phase saumure saturée + dihydrate ;
- un calcul 12, 13 (par l'unité 20) de la température T_{congel} ou T_{hydrate} de formation, dans la solution 2, de glace ou d'un hydrate sous forme solide pour la concentration déterminée Sal_{det}, à partir des données sur une température T_{congel} ou T_{hydrate} de formation, dans la solution, de glace ou d'un hydrate sous forme solide en fonction de la concentration de la solution.

Le procédé comprend :
- un calcul 12 (par l'unité 20) de la température T_{congel} de formation, dans la solution 2, de glace pour la concentration déterminée Sal_{det}, si la concentration déterminée Sal_{det} est inférieure ou égale à un seuil eutectique de préférence égal à 23,3 % (±0,1%) pour une concentration exprimée en pourcentage du poids de la solution, ou
- un calcul 13 (par l'unité 20) de la température T_{hydrate} de formation, dans la solution 2, d'un hydrate sous forme solide pour la concentration déterminée Sal_{det}, si la concentration déterminée Sal_{det} est supérieure au seuil eutectique de préférence égal à 23,3 % (±0,1%) pour une concentration exprimée en pourcentage du poids de la solution.

Dans les exemples des figures 3 à 8, l'hydrate est un dihydrate (dihydrate de sodium NaCl-2H₂O dite phase hydrohalite), et T_{hydrate} est en réalité la température T_{dihydrate} de formation, dans la solution, de ce dihydrate sous forme solide pour la concentration déterminée Sal_{det}.

Le procédé comprend enfin :
- un affichage 14, sur les moyens 21, de la température T_{congel} ou T_{hydrate} calculée, et/ou
- une comparaison 15 (par l'unité 20) de la température T_{congel} ou T_{hydrate} calculée avec la température mesurée Tₘₑₛ, et, si la température T_{congel} ou T_{hydrate} calculée est supérieure ou égale à la température mesurée Tₘₑₛ, une génération 16 (par les moyens 22) d'une alarme 18 visuelle ou sonore qui permet de prévenir de la formation de glace ou d'hydrate.

On va maintenant décrire quelques exemples pratiques de ce premier mode de réalisation de dispositif 1 et procédé selon l'invention.

### Exemple 1 (figures 3, 7 et 8):

Etape 4 : la conductivité Cₘₑₛ est 145,8 mS/cm
Etape 3 : la température Tₘₑₛ est égale à 5°C.
Etape 10 : la concentration Sal de NaCl maximum est donc de %Max=26,4 %.
Etape 5 : afin de déterminer la relation concentration - conductivité à la température considérée Tₘₑₛ, il faut donc prendre en compte les courbes de descente en froid de saumures dont la concentration est inférieure à 26,4%.

On utilise donc (étape 5) les couples de valeurs (3,7% ; 31,8 mS/cm), (8% ; 70,4 mS/cm),..., (25,5% ; 150,3 mS/cm) afin de déterminer la relation concentration - conductivité à la température considérée Tₘₑₛ.

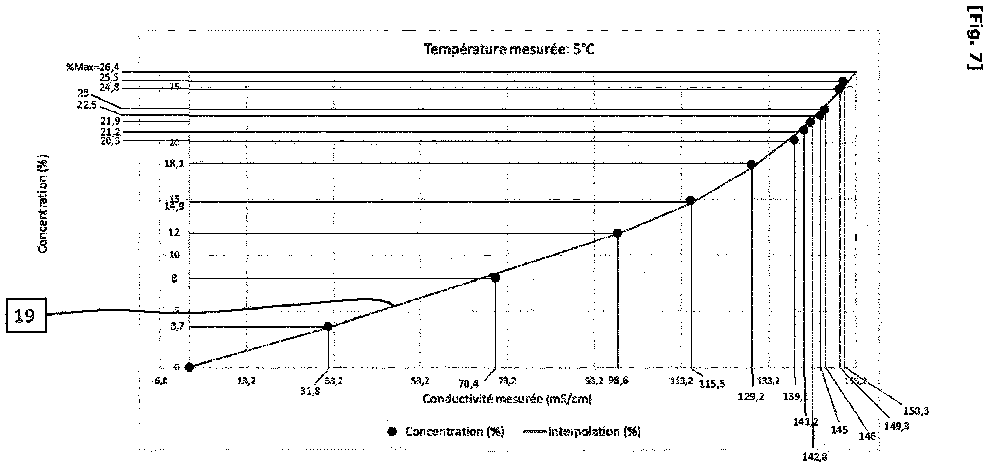

L'interpolation, illustrée sur la figure 7, est réalisée sur ces couples de valeurs afin de déterminer la relation entre concentration Sal de NaCl et conductivité C à la température Tₘₑₛ de 5°C.

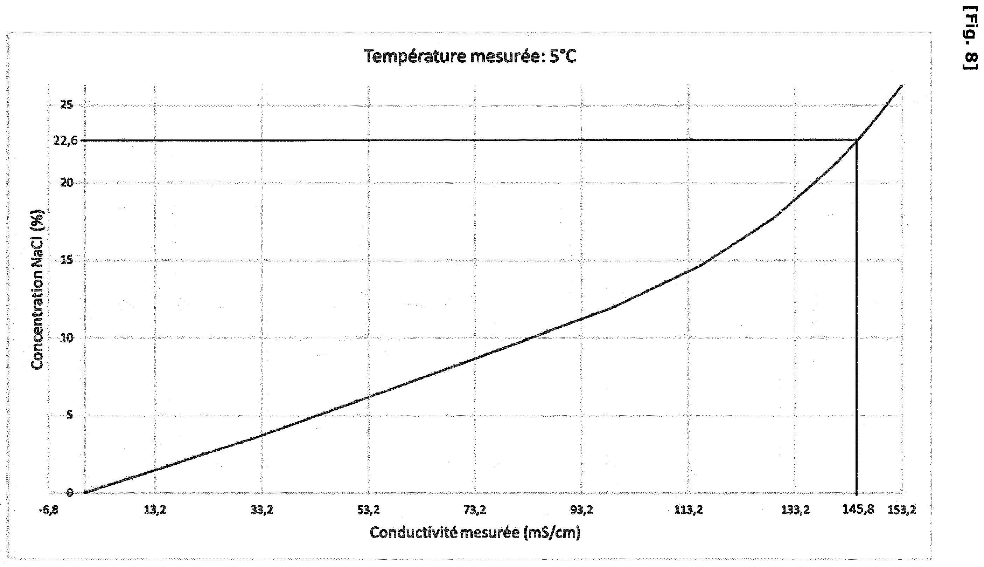

Etape 6 : l'interpolation permet ensuite (figure 8) de déduire la concentration de sel Sal_{det}.

Etape 12 : La concentration de la saumure Sal_{det} est donc 22,6% et sa température de congélation T_{congel}=-19,9°C peut ensuite être calculée.

Etape 16 : si la température de la saumure Tₘₑₛ venait à descendre à une valeur inférieure ou égale à cette température de congélation, alors une alarme est déclenchée.

### Exemple 2:

Si Tₘₑₛ = -17,7 °C
Et Cₘₑₛ = 68,4 mS/cm
Alors l'invention renvoie une concentration max de NaCl %Max=23,7%.

Les courbes de descente en température des saumures suivantes sont utilisées pour générer une courbe équivalente à la figure 7 mais pour une température de -17,7 °C : 3,7%, 8%,12%, 14,9%, 18,1%, 20,3%, 21,2%, 21,9%, 22,5%, 23%.

Une interpolation est ensuite réalisée afin d'obtenir la relation entre conductivité mesurée à la température de -17,7°C et la concentration en NaCl.

On utilise ensuite cette relation (équivalent de la figure 8) afin de déterminer la concentration de NaCl en considérant la valeur de conductivité de 68,4 mS/cm.

La valeur de concentration de NaCl obtenue est Sal_{det}=21,5%
La température de congélation de la saumure est ensuite déterminée à partir de cette concentration et on obtient une température de congélation de T_{congel}=-18,4°C.

### Exemple 3:

Les données proviennent de la même saumure que l'exemple 2.
Si Tₘₑₛ = -18,6°C
Et Cₘₑₛ = 65,9 mS/cm

Alors on obtient :
- une concentration max de NaCl de %Max=23,6%
- une concentration de NaCl de Sal_{det}=21,5%
- une température de congélation de T_{congel}= -18,4°C

Étant donné que la température Tₘₑₛ est inférieure à la température de congélation T_{congel}, une alarme est déclenchée afin d'alerter sur la formation de glace dans la saumure.

### Exemple 4:

Si Tₘₑₛ = 25 °C
Et Cₘₑₛ = 240,6 mS/cm

On obtient :
%Max= 26,6%
Sal_{det} = 25,2%
T_{dihydrate} = -6,2 °C

### Exemple 5:

Si Tₘₑₛ = -5 °C
Et Cₘₑₛ = 111 mS/cm

On obtient :
%Max = 25,5 %
Sal_{det} = 25,3 %
T_{dihydrate} = -6,4 °C

### Exemple 6:

Si Tₘₑₛ = -8 °C
Et Cₘₑₛ = 100,4 mS/cm

On obtient :
%Max = 25%
Sal_{det} = 25%
T_{dihydrate} = -8 °C
Et on génère une alarme formation de dihydrate

En outre, un premier mode de réalisation de bateau ou navire ou usine selon l'invention non illustré est équipé du premier mode de réalisation de dispositif selon l'invention et/ou met en œuvre le premier mode de réalisation de procédé selon l'invention.

Les exemples 1 à 6 ci-dessus sont mis en œuvre dans un tel bateau ou navire ou usine pour la conservation de poissons (illustrés en Figure 1). La solution est donc un mélange de saumure (eau + NaCl (et éventuels autres sels)) avec différents éléments organiques (sang de poisson, écailles, etc.) et autres éléments ou polluants que l'on peut trouver dans de l'eau de mer. Cette solution est typiquement de l'eau de mer dans laquelle on a rajouté du NaCl et des déchets organiques (de poissons ou autre).

Bien sûr, l'invention n'est pas limitée aux exemples qui viennent d'être décrits et de nombreux aménagements peuvent être apportés à ces exemples sans sortir du cadre de l'invention.

On pourrait par exemple considérer :
- comme autre soluté : un sel et/ou au moins un sel parmi NaCl, NaOH, HCl, CaCl₂, (ou autres, la liste précédente n'étant que des exemples) ou un mélange de ceux-ci, et/ou
- la solution peut comprendre comme solvant une saumure, de l'eau, de l'alcool, un hydrocarbure liquide, (ou autres, la liste précédente n'étant que des exemples) ou un mélange de ceux-ci ; ce solvant comprend de préférence au moins 80% en masse d'eau, de préférence au moins 90% en masse d'eau ; et/ou
- comme on le comprendra à la vue des exemples précédents, la solution peut aussi comprendre différents éléments organiques, typiquement en provenance de poissons (sang, écailles, etc.) ou d'autres éléments alimentaires que l'on cherche à conserver, et/ou
- comme on le comprendra à la vue des exemples précédents, la solution peut aussi comprendre différents éléments complémentaires, comme par exemple du sable (en provenance de coquilles Saint Jacques ou autres coquillages), des algues, du plancton, etc.

On remarque que le seuil eutectique (mémorisé et/ou stocké et/ou utilisé par les moyens 20 pour la mise en œuvre du procédé précédemment décrit) peut être égal à 23,3 %±0,1% pour une concentration exprimée en pourcentage du poids de la solution.

Bien entendu, les différentes caractéristiques, formes, variantes et modes de réalisation de l'invention peuvent être associées les unes avec les autres selon diverses combinaisons dans la mesure où elles ne sont pas incompatibles ou exclusives les unes des autres. En particulier toutes les variantes et modes de réalisation décrits précédemment sont combinables entre eux.

## Revendications

1. Procédé de mesure d'une concentration d'au moins un sel et/ou d'au moins un soluté dans une solution (2), comprenant les étapes suivantes :
- un stockage de données (7) reliant la concentration Sal de la solution avec la conductivité électrique C de la solution et la température T de la solution,
- une mesure (3) de température Tₘₑₛ de la solution,
- une mesure (4) de conductivité électrique Cₘₑₛ de la solution,
le couple (17) Tₘₑₛ, Cₘₑₛ de la mesure de température et de la mesure de conductivité électrique n'étant pas compris dans les données stockées (7) reliant la concentration Sal avec la conductivité électrique C et la température T de la solution,
le procédé comprenant en outre :
- une interpolation (5), en fonction des données stockées (7) reliant la concentration Sal avec la conductivité électrique C et la température T de la solution:
∘ d'une loi, qu'on appelle dans la présente revendication « loi interpolée (19) », reliant la concentration de la solution en fonction de la conductivité électrique de la solution ou inversement, pour une valeur fixe de la température de la solution égale à la température mesurée Tₘₑₛ et/ou
∘ d'une loi, qu'on appelle dans la présente revendication « loi interpolée », reliant la concentration de la solution en fonction de la température de la solution ou inversement, pour une valeur fixe de la conductivité électrique de la solution égale à la conductivité mesurée Cₘₑₛ
- une détermination (6) de la concentration Sal_{det} de la solution en fonction de cette loi interpolée et de la conductivité électrique mesurée Cₘₑₛ et/ou de la température mesurée Tₘₑₛ.

2. Procédé selon la revendication 1, **caractérisé en ce que** les données stockées (7) reliant la concentration de la solution avec la conductivité électrique et la température de la solution comprennent, pour un nombre fini de valeurs de concentration, des lois (7, 701-713) stockées, chaque loi stockée reliant température de la solution et conductivité électrique de la solution à valeur fixe de concentration de la solution.

3. Procédé selon la revendication 2, **caractérisé en ce que** le nombre fini de valeurs de concentration comprend au moins 3, de préférence au moins 10 valeurs de concentration distinctes.

4. Procédé selon la revendication 2 ou 3, **caractérisé en ce que** le nombre fini de valeurs de concentration comprend au moins une valeur inférieure à un seuil eutectique et/ou comprend au moins une valeur supérieure au seuil eutectique.

5. Procédé selon l'une quelconque des revendications 2 à 4, **caractérisé en ce que** le nombre fini de valeurs de concentration sont des valeurs successives de concentration distantes de moins de 6% pour une concentration exprimée en pourcentage du poids de la solution.

6. Procédé selon l'une quelconque des revendications 2 à 5, **caractérisé en ce que** le nombre fini de valeurs de concentration comprend au moins 5 valeurs de concentration distinctes supérieures à 20% et dont les valeurs successives de concentration sont distantes de moins de 1%, pour une concentration exprimée en pourcentage du poids de la solution.

7. Procédé selon l'une quelconque des revendications 2 à 6, **caractérisé en ce que** chaque loi stockée (7) est une loi polynomiale.

8. Procédé selon l'une quelconque des revendications précédentes, **caractérisé en ce que** la mesure de conductivité électrique (4) comprend une mesure de conductivité électrique par induction, comprenant un passage d'un premier courant électrique alternatif dans une première bobine conductrice (8) induisant un deuxième courant électrique dans la solution (2), ce deuxième courant électrique dans la solution générant un troisième courant électrique dans une deuxième bobine (9).

9. Procédé selon l'une quelconque des revendications précédentes, **caractérisé en ce qu'**il comprend :
- un stockage de données sur une limite maximum de concentration de la solution en fonction de la température de la solution, et
- un calcul (10) de la limite maximum de concentration de la solution pour la température mesurée Tₘₑₛ, à partir des données sur une limite maximum de concentration de la solution en fonction de la température.

10. Procédé selon la revendication 9, **caractérisé en ce que**, si la détermination de la concentration de la solution donne une valeur supérieure à la limite maximum de concentration de la solution pour la température mesurée, la détermination de la concentration de la solution est corrigée (11) comme étant égale à la limite maximum de concentration de la solution pour la température mesurée.

11. Procédé selon l'une quelconque des revendications précédentes, **caractérisé en ce qu'**il comprend :
- un stockage de données sur une température T_{congel} ou T_{hydrate} de formation, dans la solution, de glace ou d'un hydrate, de préférence d'un dihydrate, sous forme solide en fonction de la concentration de la solution,
- un calcul (12, 13) de la température T_{congel} ou T_{hydrate} de formation, dans la solution (2), de glace ou d'un hydrate sous forme solide pour la concentration déterminée Sal_{det}, à partir des données sur une température T_{congel} ou T_{hydrate} de formation, dans la solution, de glace ou d'un hydrate sous forme solide en fonction de la concentration de la solution.

12. Procédé selon la revendication 11, **caractérisé en ce qu'**il comprend :
- un calcul (12) de la température T_{congel} de formation, dans la solution, de glace pour la concentration déterminée Sal_{det}, si la concentration déterminée Sal_{det} est inférieure ou égale à un seuil eutectique, ou
- un calcul (13) de la température T_{hydrate} de formation, dans la solution, d'un hydrate sous forme solide pour la concentration déterminée Sal_{det}, si la concentration déterminée Sal_{det} est supérieure au seuil eutectique.

13. Procédé selon la revendication 11 ou 12, **caractérisé en ce qu'**il comprend :
- un affichage (14) de la température T_{congel} ou T_{hydrate} calculée, et/ou
- une comparaison (15) de la température T_{congel} ou T_{hydrate} calculée avec la température mesurée Tₘₑₛ, et, si la température T_{congel} ou T_{hydrate} calculée est supérieure ou égale à la température mesurée Tₘₑₛ, une génération (16) d'une alarme (18) visuelle ou sonore.

14. Procédé selon l'une quelconque des revendications précédentes, **caractérisé en ce que** la solution est ou comprend une saumure.

15. Procédé selon l'une quelconque des revendications précédentes, **caractérisé en ce que** le sel est ou comprend du chlorure de sodium.

16. Procédé selon l'une quelconque des revendications précédentes considérée comme dépendante de la revendication 4 ou 12, **caractérisé en ce que** le seuil eutectique est égal à 23,3 %±0,1% pour une concentration exprimée en pourcentage du poids de la solution.

17. Dispositif de mesure d'une concentration d'au moins un sel et/ou d'au moins un soluté dans solution (2), comprenant :
- des moyens agencés et/ou programmés pour stocker des données (7) reliant la concentration Sal de la solution avec la conductivité électrique C de la solution et la température T de la solution,
- des moyens de mesure (3) de température Tₘₑₛ de la solution,
- des moyens de mesure (4) de conductivité électrique Cₘₑₛ de la solution, le couple (17) Tₘₑₛ, Cₘₑₛ de la mesure de température et de la mesure de conductivité électrique n'étant pas compris dans les données stockées (7) reliant la concentration Sal avec la conductivité électrique C et la température T de la solution,
le dispositif comprenant en outre :
- des moyens agencés et/ou programmés pour interpoler (5), en fonction des données stockées (7) reliant la concentration Sal avec la conductivité électrique C et la température T de la solution:
∘ une loi, qu'on appelle dans la présente revendication «loi interpolée (19) », reliant la concentration de la solution en fonction de la conductivité électrique de la solution ou inversement, pour une valeur fixe de la température de la solution égale à la température mesurée Tₘₑₛ et/ou
∘ une loi, qu'on appelle dans la présente revendication «loi interpolée (19) », reliant la concentration de la solution en fonction de la température de la solution ou inversement, pour une valeur fixe de la conductivité électrique de la solution égale à la conductivité mesurée Cₘₑₛ
- des moyens agencés et/ou programmés pour déterminer (6) la concentration Sal_{det} de la solution en fonction de cette loi interpolée et de la conductivité électrique mesurée Cₘₑₛ et/ou de la température mesurée Tₘₑₛ.

18. Bateau ou navire ou installation à terre équipé d'un dispositif selon la revendication 17 ou mettant en œuvre le procédé selon l'une quelconque des revendications 1 à 16.

## Patentansprüche

1. Verfahren zur Messung einer Konzentration von wenigstens einem Salz und/oder von wenigstens einem gelösten Stoff in einer Lösung (2), umfassend die folgenden Schritte:
- eine Speicherung von Daten (7), die die Konzentration Sal der Lösung mit der elektrischen Leitfähigkeit C der Lösung und der Temperatur T der Lösung in Beziehung setzen,
- eine Messung (3) der Temperatur Tₘₑₛ der Lösung,
- eine Messung (4) der elektrischen Leitfähigkeit Cₘₑₛ der Lösung, wobei das Paar (17) Tₘₑₛ, Cₘₑₛ der Messung der Temperatur und der Messung der elektrischen Leitfähigkeit nicht in den gespeicherten Daten (7), die die Konzentration Sal mit der elektrischen Leitfähigkeit C und der Temperatur T der Lösung in Beziehung setzen, enthalten ist,
wobei das Verfahren weiterhin umfasst:
- in Abhängigkeit von den gespeicherten Daten (7), die die Konzentration Sal mit der elektrischen Leitfähigkeit C und der Temperatur T der Lösung in Beziehung setzen, eine Interpolation (5):
• eines Zusammenhangs, der im vorliegenden Anspruch "interpolierter Zusammenhang (19)" genannt wird und die Konzentration der Lösung in Abhängigkeit von der elektrischen Leitfähigkeit der Lösung oder umgekehrt für einen festen Wert der Temperatur der Lösung, die gleich der gemessenen Temperatur Tₘₑₛ ist, miteinander in Beziehung setzt, und/oder
• eines Zusammenhangs, der im vorliegenden Anspruch "interpolierter Zusammenhang" genannt wird und die Konzentration der Lösung in Abhängigkeit von der Temperatur der Lösung oder umgekehrt für einen festen Wert der elektrischen Leitfähigkeit der Lösung, die gleich der gemessenen elektrischen Leitfähigkeit Cₘₑₛ ist, miteinander in Beziehung setzt,
- eine Bestimmung (6) der Konzentration Sal_{det} der Lösung anhand dieses interpolierten Zusammenhangs und anhand der gemessenen elektrischen Leitfähigkeit Cₘₑₛ und/oder der gemessenen Temperatur Tₘₑₛ.

2. Verfahren gemäß Anspruch 1, **dadurch gekennzeichnet, dass** die gespeicherten Daten (7), die die Konzentration der Lösung mit der elektrischen Leitfähigkeit und der Temperatur der Lösung in Beziehung setzen, für eine endliche Anzahl von Konzentrationswerten gespeicherte Zusammenhänge (7, 701-713) umfassen, wobei jeder gespeicherte Zusammenhang die Temperatur der Lösung und die elektrische Leitfähigkeit der Lösung mit einem festen Wert der Konzentration der Lösung in Beziehung setzt.

3. Verfahren gemäß Anspruch 2, **dadurch gekennzeichnet, dass** die endliche Anzahl von Konzentrationswerten wenigstens 3, vorzugsweise wenigstens 10, unterschiedliche Konzentrationswerte umfasst.

4. Verfahren gemäß Anspruch 2 oder 3, **dadurch gekennzeichnet, dass** die endliche Anzahl von Konzentrationswerten wenigstens einen Wert umfasst, der unterhalb eines eutektischen Punkts liegt, und/oder wenigstens einen Wert umfasst, der oberhalb eines eutektischen Punkts liegt.

5. Verfahren gemäß einem der Ansprüche 2 bis 4, **dadurch gekennzeichnet, dass** es sich bei der endlichen Anzahl von Konzentrationswerten um aufeinanderfolgende Konzentrationswerte handelt, die weniger als 6% voneinander entfernt sind, wenn die Konzentration als Prozentanteil des Gewichts der Lösung angegeben ist.

6. Verfahren gemäß einem der Ansprüche 2 bis 5, **dadurch gekennzeichnet, dass** die endliche Anzahl von Konzentrationswerten wenigstens 3 unterschiedliche Konzentrationswerte umfasst, die oberhalb von 20% liegen und deren aufeinanderfolgende Konzentrationswerte weniger als 1% voneinander entfernt sind, wenn die Konzentration als Prozentanteil des Gewichts der Lösung angegeben ist.

7. Verfahren gemäß einem der Ansprüche 2 bis 6, **dadurch gekennzeichnet, dass** jeder gespeicherte Zusammenhang (7) ein polynomialer Zusammenhang ist.

8. Verfahren gemäß einem der vorstehenden Ansprüche, **dadurch gekennzeichnet, dass** die Messung der elektrischen Leitfähigkeit (4) eine Messung der elektrischen Leitfähigkeit durch Induktion umfasst, die das Fließen eines ersten elektrischen Wechselstroms durch eine erste Spule (8) umfasst, der in der Lösung (2) einen zweiten elektrischen Strom induziert, wobei dieser zweite elektrische Strom in der Lösung einen dritten elektrischen Strom in einer zweiten Spule (9) erzeugt.

9. Verfahren gemäß einem der vorstehenden Ansprüche, **dadurch gekennzeichnet, dass** es Folgendes umfasst:
- eine Speicherung von Daten über eine maximale Konzentrationsgrenze der Lösung in Abhängigkeit von der Temperatur der Lösung und
- eine Berechnung (10) der maximalen Konzentrationsgrenze der Lösung für die gemessene Temperatur Tₘₑₛ ausgehend von den Daten über eine maximale Konzentrationsgrenze der Lösung in Abhängigkeit von der Temperatur.

10. Verfahren gemäß Anspruch 9, **dadurch gekennzeichnet, dass** dann, wenn die Bestimmung der Konzentration der Lösung einen Wert oberhalb der maximalen Konzentrationsgrenze der Lösung für die gemessene Temperatur ergibt, die Bestimmung der Konzentration der Lösung so korrigiert wird (11), dass sie gleich der maximalen Konzentrationsgrenze der Lösung für die gemessene Temperatur wird.

11. Verfahren gemäß einem der vorstehenden Ansprüche, **dadurch gekennzeichnet, dass** es Folgendes umfasst:
- eine Speicherung von Daten über eine Temperatur T_{congel} oder T_{hydrat} der Bildung von Eis oder eines Hydrats, vorzugsweise eines Dihydrats, in fester Form in der Lösung in Abhängigkeit von der Konzentration der Lösung,
- eine Berechnung (12, 13) der Temperatur T_{congel} oder T_{hydrat} der Bildung von Eis oder eines Hydrats in fester Form in der Lösung (2) für die bestimmte Konzentration Sal_{det} ausgehend von den Daten über eine Temperatur T_{congel} oder T_{hydrat} der Bildung von Eis oder eines Hydrats in fester Form in der Lösung in Abhängigkeit von der Konzentration der Lösung.

12. Verfahren nach Anspruch 11, **dadurch gekennzeichnet, dass** es Folgendes umfasst:
- eine Berechnung (12) der Temperatur T_{congel} der Bildung von Eis in der Lösung für die bestimmte Konzentration Sal_{det}, wenn die bestimmte Konzentration Sal_{det} kleiner oder gleich einem eutektischen Punkt ist, oder
- eine Berechnung (13) der Temperatur T_{hydrat} der Bildung eines Hydrats in fester Form in der Lösung für die bestimmte Konzentration Sal_{det}, wenn die bestimmte Konzentration Sal_{det} größer als der eutektische Punkt ist.

13. Verfahren gemäß Anspruch 11 oder 12, **dadurch gekennzeichnet, dass** es Folgendes umfasst:
- eine Anzeige (14) der berechneten Temperatur T_{congel} oder T_{hydrat} und/oder
- einen Vergleich (15) der berechneten Temperatur T_{congel} oder T_{hydrat} mit der gemessenen Temperatur Tₘₑₛ, und wenn die berechnete Temperatur T_{congel} oder T_{hydrat} größer oder gleich der gemessenen Temperatur Tₘₑₛ ist, eine Erzeugung (16) eines visuellen oder auditiven Alarms (18).

14. Verfahren gemäß einem der vorstehenden Ansprüche, **dadurch gekennzeichnet, dass** die Lösung eine Salzlösung ist oder umfasst.

15. Verfahren gemäß einem der vorstehenden Ansprüche, **dadurch gekennzeichnet, dass** das Salz Natriumchlorid ist oder umfasst.

16. Verfahren gemäß einem der vorstehenden Ansprüche, der als von Anspruch 4 oder 12 abhängig angesehen wird, **dadurch gekennzeichnet, dass** der eutektische Punkt gleich 23,3% ± 0,1% ist, wenn die Konzentration als Prozentanteil des Gewichts der Lösung angegeben ist.

17. Vorrichtung zur Messung einer Konzentration von wenigstens einem Salz und/oder von wenigstens einem gelösten Stoff in einer Lösung (2), umfassend:
- Einrichtungen, die zum Speichern der Daten (7), die die Konzentration Sal der Lösung mit der elektrischen Leitfähigkeit C der Lösung und der Temperatur T der Lösung in Beziehung setzen, gestaltet und/oder programmiert sind,
- Einrichtungen zur Messung (3) der Temperatur Tₘₑₛ der Lösung,
- Einrichtungen zur Messung (4) der elektrischen Leitfähigkeit Cₘₑₛ der Lösung, wobei das Paar (17) Tₘₑₛ, Cₘₑₛ der Messung der Temperatur und der Messung der elektrischen Leitfähigkeit nicht in den gespeicherten Daten (7), die die Konzentration Sal mit der elektrischen Leitfähigkeit C und der Temperatur T der Lösung in Beziehung setzen, enthalten ist,
wobei die Vorrichtung weiterhin Folgendes umfasst:
- Einrichtungen, die in Abhängigkeit von den gespeicherten Daten (7), die die Konzentration Sal mit der elektrischen Leitfähigkeit C und der Temperatur T der Lösung in Beziehung setzen, gestaltet und/oder programmiert sind zur Interpolation (5):
• eines Zusammenhangs, der im vorliegenden Anspruch "interpolierter Zusammenhang (19)" genannt wird und die Konzentration der Lösung in Abhängigkeit von der elektrischen Leitfähigkeit der Lösung oder umgekehrt für einen festen Wert der Temperatur der Lösung, die gleich der gemessenen Temperatur Tₘₑₛ ist, miteinander in Beziehung setzt, und/oder
• eines Zusammenhangs, der im vorliegenden Anspruch "interpolierter Zusammenhang (19)" genannt wird und die Konzentration der Lösung in Abhängigkeit von der Temperatur der Lösung oder umgekehrt für einen festen Wert der elektrischen Leitfähigkeit der Lösung, die gleich der gemessenen elektrischen Leitfähigkeit Cₘₑₛ ist, miteinander in Beziehung setzt,
- Einrichtungen, die zur Bestimmung (6) der Konzentration Sal_{det} der Lösung anhand dieses interpolierten Zusammenhangs und anhand der gemessenen elektrischen Leitfähigkeit Cₘₑₛ und/oder der gemessenen Temperatur Tₘₑₛ gestaltet und/oder programmiert sind.

18. Schiff oder Boot oder Anlage an Land, das oder die mit einer Vorrichtung gemäß Anspruch 17 ausgestattet ist oder das Verfahren gemäß einem der Ansprüche 1 bis 16 ausführt.

## Claims

1. Method for measuring a concentration of at least one salt and/or of at least one solute in a solution (2), comprising the following steps:
- storing data (7) linking the concentration Sal of the solution with the electrical conductivity C of the solution and the temperature T of the solution,
- measuring (3) temperature Tmeas of the solution,
- measuring (4) electrical conductivity Cmeas of the solution,
the pair (17) Tmeas, Cmeas of the temperature measurement and the electrical conductivity measurement not being comprised in the stored data (7) linking the concentration Sal with the electrical conductivity C and the temperature T of the solution,
the method also comprising:
- an interpolation (5), as a function of the stored data (7) linking the concentration Sal with the electrical conductivity C and the temperature T of the solution:
∘ of a law, which is called in the present claim "interpolated law (19)", linking the concentration of the solution as a function of the electrical conductivity of the solution or vice versa, for a set value of the temperature of the solution equal to the measured temperature Tmeas and/or
∘ of a law, which is called in the present claim "interpolated law", linking the concentration of the solution as a function of the temperature of the solution or vice versa, for a set value of the electrical conductivity of the solution equal to the measured conductivity Cmeas
- a determination (6) of the concentration Saldet of the solution as a function of this interpolated law and of the measured electrical conductivity Cmeas and/or of the measured temperature Tmeas.

2. Method according to claim 1, **characterized in that** the stored data (7) linking the concentration of the solution with the electrical conductivity and the temperature of the solution comprise, for a finite number of concentration values, stored laws (7, 701-713), each stored law linking temperature of the solution and electrical conductivity of the solution to a set concentration value of the solution.

3. Method according to claim 2, **characterized in that** the finite number of concentration values comprises at least 3, preferably at least 10 distinct concentration values.

4. Method according to claim 2 or 3, **characterized in that** the finite number of concentration values comprises at least one value less than a eutectic threshold and/or comprises at least one value greater than the eutectic threshold.

5. Method according to any one of claims 2 to 4, **characterized in that** the finite number of concentration values are successive concentration values separated by less than 6% for a concentration expressed as a percentage of the weight of the solution.

6. Method according to any one of claims 2 to 5, **characterized in that** the finite number of concentration values comprises at least 5 distinct concentration values greater than 20%, the successive concentration values of which are separated by less than 1%, for a concentration expressed as a percentage of the weight of the solution.

7. Method according to any one of claims 2 to 6, **characterized in that** each stored law (7) is a polynomial law.

8. Method according to any one of the preceding claims, **characterized in that** the measurement of electrical conductivity (4) comprises a measurement of electrical conductivity by induction, comprising passing a first alternating electric current through a first conductive coil (8) inducing a second electric current in the solution (2), this second electric current in the solution generating a third electric current in a second coil (9).

9. Method according to any one of the preceding claims, **characterized in that** it comprises:
- storing data on a maximum limit of concentration of the solution as a function of the temperature of the solution, and
- calculating (10) the maximum limit of concentration of the solution for the measured temperature Tmeas, based on the data on a maximum limit of concentration of the solution as a function of the temperature.

10. Method according to claim 9, **characterized in that**, if the determination of the concentration of the solution gives a value greater than the maximum limit of concentration of the solution for the measured temperature, the determination of the concentration of the solution is corrected (11) as being equal to the maximum limit of concentration of the solution for the measured temperature.

11. Method according to any one of the preceding claims, **characterized in that** it comprises:
- storing data on a temperature Tfreeze or Thydrate of formation, in the solution, of ice or of a hydrate, preferably of a dihydrate, in solid form as a function of the concentration of the solution,
- calculating (12, 13) the temperature Tfreeze or Thydrate of formation, in the solution (2), of ice or of a hydrate in solid form for the determined concentration Saldet, based on the data on a temperature Tfreeze or Thydrate of formation, in the solution, of ice or of a hydrate in solid form as a function of the concentration of the solution.

12. Method according to claim 11, **characterized in that** it comprises:
- calculating (12) the temperature Tfreeze of formation, in the solution, of ice for the determined concentration Saldet, if the determined concentration Saldet is less than or equal to a eutectic threshold, or
- calculating (13) the temperature Thydrate of formation, in the solution, of a hydrate in solid form for the determined concentration Saldet, if the determined concentration Saldet is greater than the eutectic threshold.

13. Method according to claim 11 or 12, **characterized in that** it comprises:
- displaying (14) the calculated temperature Tfreeze or Thydrate, and/or
- comparing (15) the calculated temperature Tfreeze or Thydrate with the measured temperature Tmeas, and, if the calculated temperature Tfreeze or Thydrate is greater than or equal to the measured temperature Tmeas, generating (16) a visual or audible alarm (18).

14. Method according to any one of the preceding claims, **characterized in that** the solution is or comprises a brine.

15. Method according to any one of the preceding claims, **characterized in that** the salt is or comprises sodium chloride.

16. Method according to any one of the preceding claims considered as dependent on claim 4 or 12, **characterized in that** the eutectic threshold is equal to 23.3% ±0.1% for a concentration expressed as a percentage of the weight of the solution.

17. Device for measuring a concentration of at least one salt and/or of at least one solute in solution (2), comprising:
- means arranged and/or programmed to store data (7) linking the concentration Sal of the solution with the electrical conductivity C of the solution and the temperature T of the solution,
- means for measuring (3) temperature Tmeas of the solution,
- means for measuring (4) electrical conductivity Cmeas of the solution,
the pair (17) Tmeas, Cmeas of the temperature measurement and the electrical conductivity measurement not being comprised in the stored data (7) linking the concentration Sal with the electrical conductivity C and the temperature T of the solution,
the device also comprising:
- means arranged and/or programmed to interpolate (5), as a function of the stored data (7) linking the concentration Sal with the electrical conductivity C and the temperature T of the solution:
∘ a law, which is called in the present claim "interpolated law (19)", linking the concentration of the solution as a function of the electrical conductivity of the solution or vice versa, for a set value of the temperature of the solution equal to the measured temperature Tmeas and/or
∘ a law, which is called in the present claim "interpolated law (19)", linking the concentration of the solution as a function of the temperature of the solution or vice versa, for a set value of the electrical conductivity of the solution equal to the measured conductivity Cmeas
- means arranged and/or programmed to determine (6) the concentration Saldet of the solution as a function of this interpolated law and of the measured electrical conductivity Cmeas and/or of the measured temperature Tmeas.

18. Boat or ship or installation on land equipped with a device according to claim 17 or implementing the method according to any one of claims 1 to 16.
